# EUROPÄISCHE PATENTSCHRIFT

(11) **EP 1 994 953 B1**
(45) Veröffentlichungstag und Bekanntmachung des Hinweises auf die Patenterteilung: **19.08.2009**
(21) Anmeldenummer: 08008848.7
(22) Anmeldetag: 13.05.2008
(51) Int. Cl.: A61M 21/02, G10L 11/04

(54) **Verfahren zur individuellen und gezielten Klangbeaufschlagung einer Person und Vorrichtung zur Durchführung des Verfahrens**
Method for individualized and targeted presentation of a sound to a person and device for executing the method
Procédé de rendu sonore individuel et ciblé sur une personne et dispositif d'exécution du procédé

(30) Priorität: 22.05.2007 DE 102007023683
(43) Veröffentlichungstag der Anmeldung: 26.11.2008
(73) Patentinhaber: Cramer, Annette, 80639 München (DE)
(72) Erfinder: Cramer, Annette, 80639 München (DE)
(74) Vertreter: Kramer - Barske - Schmidtchen

(56) Entgegenhaltungen:
- WO-A-01/06494
- WO-A-96/15823
- DE-A1- 10 329 166
- US-A- 3 760 108
- US-A- 4 388 918
- US-A- 4 755 889

## Beschreibung

Die Erfindung betrifft ein Verfahren zur individuellen und gezielten Klangbeaufschlagung einer Person, im Folgenden Sensophonie-Verfahren, und eine Vorrichtung zum Durchführen des Verfahrens.

Im menschlichen Körper stehen sämtliche Muskeln über Muskelschlingen mit dem Kehlkopf in Verbindung. Aus diesem Grunde wirken sich Verspannungen auch auf den Kehlkopf aus. Infolgedessen wird in einem angespannten Zustand mit einer höheren Stimme gesprochen als wenn der Köper entspannt bzw. müde ist.

Zum Lösen von Verspannungen wird im Bereich der Rehabilitation und Wellness unter anderem mit Klangtherapien gearbeitet. Bislang wird in diesen Bereichen aber nicht individuell auf die Verfassung eines Patienten eingegangen, sondern die Patienten werden mit beliebigen Klängen bzw. Musik behandelt, die nicht auf den individuellen Patienten angepasst waren. Dementsprechend sind die Erfolge einer derartigen Behandlung eher zufälliger Natur.

DE 299 01 078 U1 offenbart eine Vorrichtung zur Klangtherapie.

DE 297 19 376 U1 offenbart ein Musikinstrument für die Klang- bzw. Musiktherapie.

DE 94 22 455 U1 offenbart ein Saiteninstrument zur therapeutischen Behandlung.

CH 681 354 A5 offenbart eine Behandlungsliese zur Klangtherapie.

Die WO 01/06494 A1 offenbart eine Vorrichtung und ein Verfahren zum Identifizieren von Frequenz-bändern zum Berechnen von linearen Phasenverschiebungen zwischen. Datenübertragungsblöcken in einem Sprachkodierer.

Die US 4 755 889 A offenbart ein System zum Aufzeichnen und Wiedergeben von Audio- und Videosignalen.

WO 9615823 A1 offenbart eine Vorrichtung zun Therapie mit Klängen und Geräuschen. Eine Aufgabe der Erfindung ist es, ein Verfahren zur individuellen und gezielten Klangbeaufschlagung einer Person, im Folgenden Sensophonie-Verfahren, und eine Vorrichtung zum Durchführen des Verfahrens anzugeben, mit dem Klang individuell und gezielt zur Entspannung und/oder Vitalisierung des menschlichen Körpers eingesetzt werden kann.

Diese Aufgabe wird durch ein Verfahren gemäß Patentanspruch 1 und durch eine Vorrichtung gemäß Patentanspruch 8 gelöst.

Weitere vorteilhafte Ausführungsformen der Erfindung sind in den abhängigen Ansprüchen angegeben.

Der Erfindung liegt die Idee zugrunde, einer bestimmten Person mittels gezielter KJangbeaufschlagung in Form von einer bestimmten charakteristischen Frequenz, die ihrer eigenen Stimme entnommen wurde und entsprechend aufbereitet wieder zugespielt wird, Entspannung; Ausgeglichenheit oder Energie zuzuführen.

Weitere Merkmale und Zweckmäßigkeiten der Erfindung ergeben sich aus der Beschreibung anhand der Figuren, von denen zeigen:
Fig. 1 eine erste Ausführungsform einer Vorrichtung zum Durchführen eines Sensophonie-Verfahrens,
Fig. 2 ein Frequenzspektrum einer aufgenommenen Frauenstimme,
Fig. 3 zeigt einen Screen-shot eines Histogramms, wie es auf einem Computerbildschirm angezeigt wird, und
Fig. 4 eine zweite Ausführungsform einer Vorrichtung zum Durchführen des Sensophonie-Verfahrens.

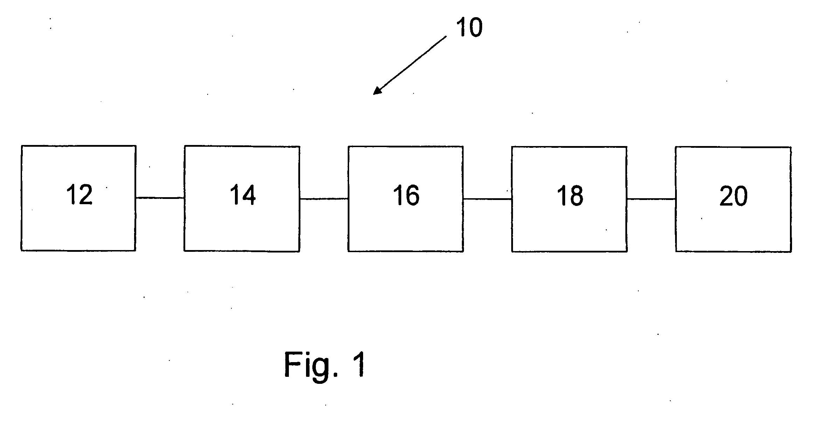

Fig. 1 zeigt eine erste Ausführungsform einer Vorrichtung 10 zum Durchführen eines Sensophonie-Verfahrens. Die Vorrichtung 10 weist eine Aufnahmeeinheit 12, eine Rechnereinheit 14, eine Analyseeinheit 16, eine Speichereinheit 18 und eine Wiedergabeeinheit 20 auf.

Die Aufnahmeeinheit 12 nimmt eine menschliche Stimme in elektronischer Form auf und ist beispielsweise ein handelsübliches Mikrophon. Bei der Aufnahme der Stimme wird in einem zeitlichen Abstand von beispielsweise 30 ms eine Fundamentalfrequenz F0 gemessen. Die Fundamentalfrequenz F0 ist die aktuelle Stimmfrequenz, welche mit der Grundschwingung der Stimme identisch ist.

Die Rechnereinheit 14 ermittelt ein Histogramm der Fundamentalfrequenz F0. In dem Histogramm wird die relative Häufigkeit einer bestimmten Fundamentalfrequenz F0 bezüglich der Anzahl aller Frequenzmessungen dargestellt. Folglich ist das Histogramm von der Länge der Sprachaufnahme unabhängig. Es sollte beachtet werden, dass das Histogramm nicht ein Frequenzspektrum des Klangs der Stimme zeigt, sondern einen Tonumfang der Sprachmelodie des jeweiligen Sprechers.

Die Analyseeinheit 16 analysiert das Histogramm und filtert drei charakteristische Frequenzbereiche mittels konventioneller Frequenzanalysetechniken aus dem Histogramm Dabei ist die Analyse darauf gerichtet, drei charakteristische Frequenzen der menschlichen Stimme der betroffenen Person zu identifizieren. Die Speichereinheit 18 speichert die drei gefilterten charakteristischen Frequenzbereiche in elektronischer Form. Die Rechnereinheit 14, die Analyseeinheit 16 und die Speichereinheit 18 sind auf einem PC installiert und werden von einem Programm angesteuert. Die Wiedergabeeinheit 20 ist geeignet, jeweils einen der drei charakteristischen Frequenzbereiche akustisch wiederzugeben und ist beispielsweise ein Kopfhörer. Dabei ist zu beachten, dass die Wiedergabe der charakteristischen Frequenzbereiche bzw. deren Töne mittels natürlicher Instrumente, wie beispielsweise Saiteninstrumente, erfolgt, so dass es sich nicht um synthetische Klänge handelt.

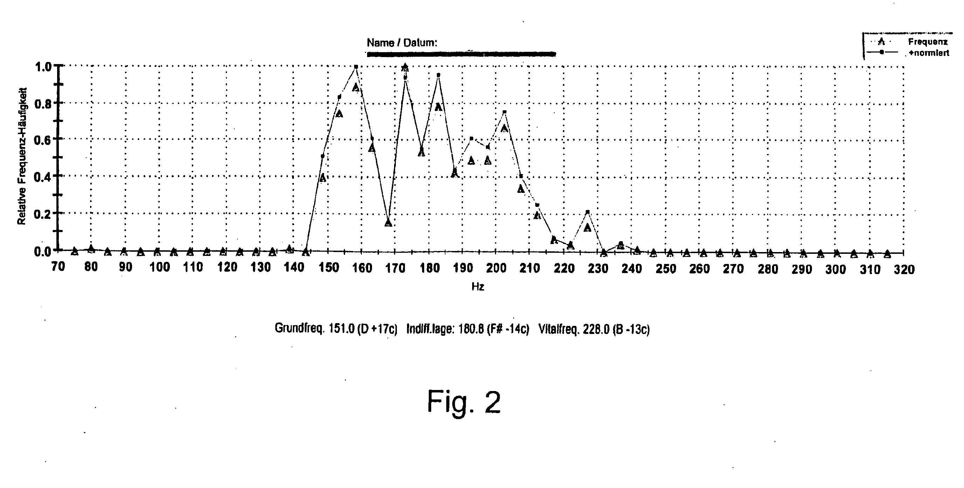

Fig. 2 zeigt ein beispielhaftes Histogramm einer aufgenommenen Frauenstimme. Es ist zu erkennen, dass das Histogramm eine Mehrzahl von Peaks mit unterschiedlichen Amplituden aufweist. Entsprechende Peaks können im Histogramm jeder menschlichen Stimme lokalisiert werden. In dem Histogramm werden die drei charakteristischen Frequenzbereiche von einer Grundfrequenz, einer Indifferenzlagefrequenz und einer Vitalfrequenz gebildet. Die Grundfrequenz, die Indifferenzlagefrequenz und die Vitalfrequenz sind in dem Histogramm jeweils voneinander beabstandet.

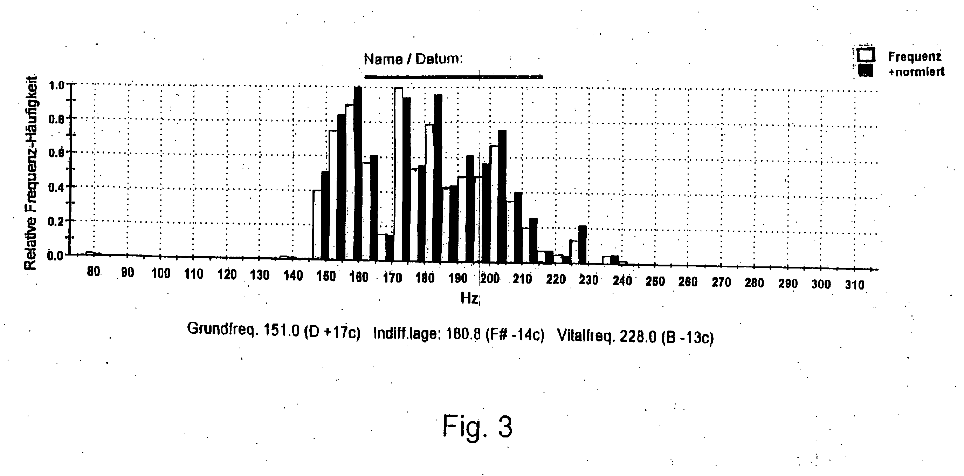

Fig. 3 zeigt einen Screen-shot des Histogramms, wie es auf einem Computerbildschirm angezeigt wird.

Die Indifferenzlagefrequenz wird anhand der Eigenschaft ermittelt, dass diese eine Frequenz ist, die während eines Sprechens am häufigsten verwendet wird. Sie kann somit durch Auswertung von zeitlich längeren Abfolgen von gesprochener Sprache dahingehend, welche Frequenz vorherrschend ist, bestimmt werden. Sie befindet sich im Wesentlichen im unteren Drittel eines individuellen Stimmumfangs. Sie ist in besonderem Maße sprecherspezifisch und abhängig von der körperlichen und mentalen Verfassung. Bei Frauen befindet sie sich in einem Frequenzbereich von 65 bis 256 Hz, bei Männern in einem Frequenzbereich von 87 bis 131 Hz und bei Kindern in einem Frequenzbereich von 250 bis 294 Hz. Die Indifferenzlagefrequenz ergibt sich aus dem Schwerpunkt des Histogramms.

Die Grundfrequenz tritt beim Sprechen zum Ende eines Satzes und bei Entspannung des menschlichen Körpers auf. Somit kann sie durch Zuordnung des gesprochenen Satzverlaufs zu der Frequenzaufzeichnung identifiziert werden, d.h. es wird die Frequenz bzw. der Ton zum Satzende hin ermittelt. Ausgehend von der Indifferenzlagefrequenz kann die Grundfrequenz auch durch statistische Auswertung des Histogramms anhand der Eigenschaft ermittelt werden, dass die Grundfrequenz im Wesentlichen um eine kleine oder große Terz niedriger als die Indifferenzlagefrequenz ist. Eine weitere Möglichkeit zum Ermitteln der Grundfrequenz besteht darin, dass sie sich in dem Histogramm in einem linken Randbereich der Frequenzhäufung befindet, so dass die erste größere Amplitude links in dem Histogramm der Grundfrequenz entspricht. Bei Frauen befindet sich die Grundfrequenz in einem Frequenzbereich von 47 bis 220 Hz, bei Männern in einem Frequenzbereich von 73 bis 110 Hz und bei Kindern in einem Frequenzbereich von 175 bis 262 Hz.

Die Vitalfrequenz tritt beim Sprechen in einem Zustand der Freude, Erregung oder Konzentration des menschlichen Köpers auf. Häufig tritt sie tritt beim Sprechen zum Ende eines Fragesatzes auf. Somit kann sie durch Zuordnung des gesprochenen Satzverlaufs zu der Frequenzaufzeichnung identifiziert werden. Die Vitalfrequenz kann auch durch statistische Auswertung des Histogramms anhand der Eigenschaft ermittelt werden, dass sie im Wesentlichen um eine große Terz oder Quarte höher als die Indifferenzlagefrequenz ist. In einem Histogramm ist diese Frequenz deutlich vorhanden, erscheint aber nicht als Frequenzspitze, wie es beispielsweise bei Schmerz- oder Schreckempfinden der Fall wäre. Eine weitere Möglichkeit zum Ermitteln der Vitalfrequenz besteht darin, dass sie sich in dem Histogramm in einem rechten Randbereich der Frequenzhäufung befindet, so dass die erste größere Amplitude rechts in dem Histogramm der Vitalfrequenz entspricht.

In dem in Fig. 2 gezeigten Histogramm liegt die Grundfrequenz bei 151,0 Hz, die Indifferenzlagefrequenz bei 180,8 Hz und die Vitalfrequenz bei 228,0 Hz. Fig. 3 zeigt einen Screen-shot des Histogramms, wie es auf einem Computerbildschirm angezeigt wird.

In Abhängigkeit von der gewünschten Wirkung wird anschließend eine der drei gefilterten Frequenzen mittels Klangbeaufschlagung mit Klängen exakt dieser Frequenz wiedergegeben. So wirkt die Grundfrequenz entspannend, schmerzlindernd bzw. schmerzlösend, schlaffördernd und fördert die Körperwahmehmung. Dabei ist sie im menschlichen Körper besonders in einem Bereich des Beckens ansprechend.

Die Indifferenzlagefrequenz wirkt ausgleichend, führt zu einem ausgeglichenen Körpertonus, fördert die Körperwahrnehmung und Stimmung ohne sich aber euphorisch auszuwirken. Dabei ist sie im menschlichen Körper besonders in Bereichen, die an das Becken angrenzen, ausgehend von dem Beckenbereich, ansprechend.

Die Vitalfrequenz wirkt tonisierend, muntermachend, konzentrationsfördernd und bringt Energie und Körperspannung. Dabei ist sie im menschlichen Körper besonders in einem Bereich der oberen Körperpartien ansprechend.

Alternativ ist es auch in Abhängigkeit der gewünschten Wirkung möglich, eine Komposition aus den drei gefilterten Frequenzen wiederzugeben.

Neben der jeweiligen Frequenz ist für die Wirkung bei einer Wiedergabe die Art der Wiedergabe entscheidend. So kann in Abhängigkeit des gewünschten Effekts und Ansprechverhaltens des Patienten die entsprechende Frequenz der Frequenzbereiche durch die Wiedergabeeinheit 20 als Klang von verschiedenen Musikinstrumenten oder durch eine Vielzahl von Musikinstrumenten wiedergegeben werden. Des Weiteren kann zusätzlich zu der wiedergegebenen Frequenz bzw. deren Ton eine weitere beliebige Melodie wiedergegeben werden. Die Wiedergabe kann auch über Lautsprecher, die einen Raum beschallen, oder durch Koppelung mit einem Medium wie beispielsweise einer Matte, Liege oder Wasser in einer Wanne erfolgen. Des Weiteren ist möglich, Lautsprecher mit Membranen zu verbinden, die beispielsweise an Gelenken der Person angeordnet werden, wodurch gezielt Orte im Körper der Person angesprochen werden können. Zur Verstärkung der Wirkung der Wiedergabe der jeweiligen Frequenzen ist es möglich, der zu erzielenden Wirkung entsprechende Aromen abzugeben.

Optional kann in dem Histogramm zusätzlich die normierte Lautstärke eines Klangs in Abhängigkeit der Fundamentalfrequenz einbezogen werden. Dabei weisen höhere Werte in der relativen Häufigkeit auf eine höhere Lautstärke bei der entsprechenden Frequenz hin. Würde die menschliche Stimme bei allen Klangwerten mit der gleichen Lautstärke geäußert werden, wären die beiden beschriebenen Histogrammarten identisch.

Die Betriebsweise der Vorrichtung 10 der ersten Ausführungsform wird im Folgenden unter Bezugnahme auf die Fig. 1 und 2 beschrieben. Zu Beginn wird die Stimme einer Person, die lediglich ein oder zwei Sätze sprechen muss, durch die Aufnahmeeinheit 12 in elektronischer Form aufgenommen. Dabei ist zu beachten, dass die Aufnahme vor jeder Behandlung erneut erfolgt, da sich die körperliche Verfassung der Person und somit die Stimme ändert. Von der aufgenommenen Stimme wird in bekannter Weise durch die Rechnereinheit 14 das Histogramm der Stimme bestimmt. Anhand der Lage von charakteristischen Amplituden werden die drei charakteristischen Frequenzbereiche durch die Analyseeinheit 16 aus dem Histogramm gefiltert.

Die drei Frequenzbereiche werden in der Speichereinheit 18 hinterlegt, so dass diese beispielsweise bei einer therapeutischen Verwendung aufgerufen und in Abhängigkeit von der Beschwerdeindikation gezielt durch die Wiedergabeeinheit 20 wiedergegeben werden können. Ist es beispielsweise bei einem Patienten erforderlich, Verspannungen zu lösen, so wird dem Patienten die Grundfrequenz seiner Stimme durch die Wiedergabeeinheit 20 wieder zugespielt. Eine Wiedergabe erfolgt üblicherweise über eine Dauer von ca. 20 Minuten, kann aber den jeweiligen Bedürfnissen bzw. Umständen angepasst werden.

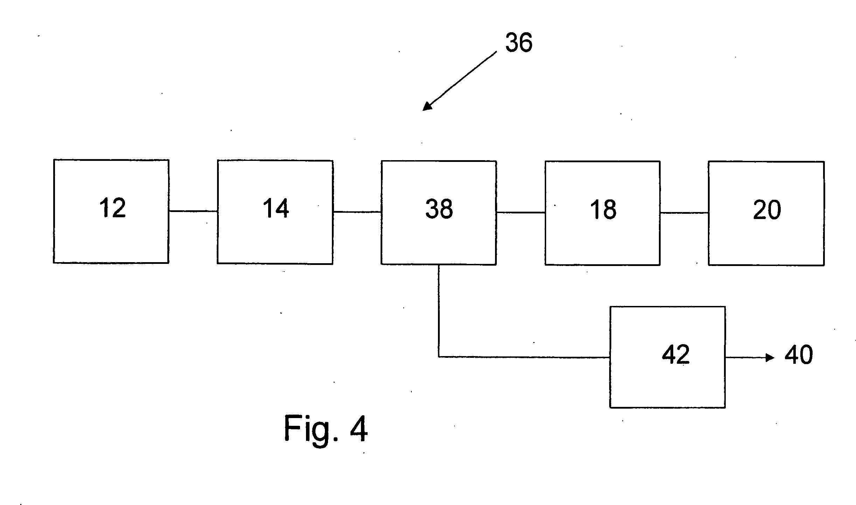

Fig. 4 zeigt eine zweite Ausführungsform der Vorrichtung zum Durchführen des Sensophonie-Verfahrens. Die Vorrichtung 36 unterscheidet sich von der ersten Ausführungsform darin, dass eine Analyseeinheit 38 den Frequenzen der drei Frequenzbereiche, die durch die Analyseeinheit 14 gefiltert wurden, Farbtöne 40 zuweist. Dabei ist die Frequenz des zugeordneten Farbtons 40 umso höher, je höher die Frequenz des entsprechenden Bereichs der Stimme ist. Das heißt, dass bei einer hohen Frequenz der Stimme blaue Farbtöne 40 zugeordnet werden und bei einer niedrigen Frequenz der Stimme rote Farbtöne 40 zugeordnet werden.

Zur Wiedergabe eines der entsprechenden Farbtöne 40 ist eine Farbwiedergabeeinheit 42 vorgesehen. Die Farbwiedergabeeinheit 42 kann beispielsweise eine handelsübliche Lampe sein, die geeignet ist, einen Raum in dem entsprechenden Farbton 40 auszuleuchten.

Die Betriebsweise der Vorrichtung 36 der zweiten Ausführungsform wird im Folgenden unter Bezugnahme auf die Fig. 4 beschrieben. Die Betriebsweise ist im Wesentlichen die gleiche wie bei der ersten Ausführungsform. Der Unterschied zu der ersten Ausführungsform liegt darin, dass bei einer therapeutischen Behandlung parallel zu der Wiedergabe des entsprechenden Frequenzbereichs eine Wiedergabe des entsprechenden Farbtons 40 erfolgt. Ist es beispielsweise bei einem Patienten erforderlich, Verspannungen zu lösen, so wird dem Patienten die Grundfrequenz seiner Stimme zugespielt und ein Behandlungsraum mit einem roten Farbton 40 durch die Farbwiedergabeeinheit 42 ausgeleuchtet.

Alle Merkmale der beschriebenen Ausführungsformen können frei kombiniert werden.

Es wird explizit betont, dass alle in der Beschreibung und/oder den Ansprüchen offenbarten Merkmale als getrennt und unabhängig voneinander zum Zweck der ursprünglichen Offenbarung unabhängig von den Merkmalskombinationen in den Ausführungsformen und/oder den Ansprüchen angesehen werden sollen. Es wird explizit festgehalten, dass alle Bereichsangaben oder Angaben von Gruppen von Einheiten jeden möglichen Zwischenwert oder Untergruppe von Einheiten zum Zweck der ursprünglichen Offenbarung offenbaren, insbesondere auch als Grenze einer Bereichsangabe. Der Schutzbereich der vorliegenden Erfindung wird durch die Ansprüche festgelegt.

### Bezugszeichenliste

- 10: Vorrichtung
- 12: Aufnahmeeinheit
- 14: Rechnereinheit
- 16: Analyseeinheit
- 18: Speichereinheit
- 20: Wiedergabeeinheit

- 36: Vorrichtung
- 38: Analyseeinheit
- 40: Farbton
- 42: Farbwiedergabeeinheit

## Patentansprüche

1. Verfahren zur individuellen und gezielten Klangbeaufschlagung einer Person, aufweisend die Schritte:
Aufnehmen einer menschlichen Stimme in elektronischer Form,
Bestimmung eines Histogramms zur Darstellung der relativen Häufigkeit einer bestimmten Fundamentalfrequenz in der aufgenommenen Stimme,
Analyse des Histogramms zum Identifizieren von drei charakteristischen Frequenzen,
Speichern der drei charakteristischen Frequenzen in elektronischer Form, Wiedergabe eines Tons mit einer der drei bestimmten charakteristischen Frequenzen in akustischer Form.

2. Verfahren nach Anspruch 1, bei dem die drei charakteristischen Frequenzen, als Amplituden in dem Histogramm der menschlichen Stimme bestimmt werden.

3. Verfahren nach Anspruch 1 oder 2, wobei die drei charakteristischen Frequenzen voneinander beabstandet sind.

4. Verfahren nach Anspruch 3, bei dem die drei charakteristischen Frequenzen als eine Grundfrequenz, eine Indifferenzlagefrequenz und eine Vitalfrequenz ausgebildet sind, wobei die Indifferenzlagefrequenz eine Frequenz aufweist, die im Wesentlichen eine kleine oder große Terz höher ist als die Frequenz der Grundfrequenz, und die Vitalfrequenz eine Frequenz aufweist, die im Wesentlichen eine große Terz oder Quarte höher ist als die Frequenz der Indifferenzlagefrequenz.

5. Verfahren nach einem der vorhergehenden Ansprüche, weiter enthaltend die Schritte: Zuordnen von entsprechenden Farbtönen (40) zu den bestimmten charakteristischen Frequenzen und
visuelle Wiedergabe des zugeordneten Farbtons (40) zusammen mit dem einen der drei charakteristischen Frequenzen.

6. Verfahren nach Anspruch 5, bei dem die Frequenzen der Farbtöne (40) höher den charakteristischen Frequenzen zugeordnet werden, desto höher die Frequenzen der charakteristischen Frequenzen sind.

7. Verfahren nach einem der vorhergehenden Ansprüche, weiter enthaltend die Schritte: Zuordnen von entsprechenden Aromen zu den bestimmten charakteristischen Frequenzen.

8. Vorrichtung (10) zum Durchführen eines Verfahrens nach einem der Ansprüche 1 bis 4, mit
einer Aufnahmeeinheit (12) zum Aufnehmen einer menschlichen Stimme,
einer Rechnereinheit (14) zum Bestimmen eines Histogramms auf S.1 der aufgezeichneten Stimme,
einer Analyseeinheit (16, 38) zum Analysieren des Histogramms zum Identifizieren von drei charakteristischen Frequenzen,
einer Speichereinheit (18) zum Speichern der drei charakteristischen Frequenzen, in elektronischer Form, und
einer Wiedergabeeinheit (20) zum Wiedergeben von einem Ton mit einer der drei charakteristischen Frequenen.

9. Vorrichtung (36) nach Anspruch 8, weiter enthaltend:
eine Analyseeinheit (38) zum Zuweisen eines Farbtons (40) zu den identifizierten charakteristischen Frequenzen, und
eine Farbwiedergabeeinheit (42) zum Wiedergeben des Farbtons (40), der der wiedergegebenen Frequenz der charakteristischen Frequenzen zugeordnet ist.

10. Vorrichtung (36) nach Anspruch 8 oder 9, weiter enthaltend:
eine Aromenwiedergabeeinheit zum Wiedergeben von wenigstens einem Aroma, das der wiedergegebenen Frequenz der charakteristischen Frequenzen zugeordnet ist.

11. Verfahren nach einem der Ansprüche 1 bis 7, bei dem die Schritte des Verfahrens als ein Programmcode formuliert sind, durch den das Verfahren auf einem Computer läuft.

## Claims

1. A method for individually and specifically applying sound to a person comprising the steps of:
recording a human voice in electronic format;
identifying a histogram for illustrating the relative occurrence of a certain fundamental frequency in the recorded voice;
analysing the histogram for identifying of three characteristic frequencies;
storing the three characteristic frequencies in electronic format;
playing back a tone having one of the three identified characteristic frequencies in acoustic format.

2. The method according to claim 1, wherein the three characteristic frequencies are identified as amplitudes in the histogram of the human voice.

3. The method according to claim 1 or 2, wherein the three characteristic frequencies are spaced apart of each other.

4. The method according to claim 3, wherein the three characteristic frequencies are formed as a basic frequency, an indifference position frequency and a vital frequency, wherein the indifference position frequency comprises a frequency being substantially a minor third or a major third higher than the frequency of the basic frequency, and the vital frequency comprises a frequency being substantially a major third or a fourth higher than the frequency of the indifference position frequency.

5. The method according to any preceding claim further comprising the steps of:
allotting corresponding colours (40) to the identified characteristic frequencies; and
visually playing back the allotted colour (40) together with the one of the three characteristic frequencies.

6. The method according to claim 5, wherein the frequencies of the colours (40) are allotted the higher to the characteristic frequencies the higher the frequencies of the characteristic frequencies are.

7. The method according to any preceding claim further comprising the steps of:
allotting corresponding flavours to the identified characteristic frequencies.

8. An apparatus (10) for carrying out the method according to one of claims 1 to 4 comprising
a recording unit (12) for recording a human voice,
a calculating unit (14) for identifying a histogram for illustrating the relative occurrence of a certain fundamental frequency in the recorded voice,
an analysing unit (16, 38) for analysing the histogram for indentifying of three characteristic frequencies,
a storing unit (18) for storing the three characteristic frequencies in electronic format, and
a playing back unit (20) for playing back a tone having one of the three characteristic frequencies.

9. The apparatus (36) according to claim 8 further comprising
an analysing unit (38) for allotting a colour (40) to the identified characteristic frequencies, and
a colour playing back unit (42) for playing back the colour (40) allotted to the played back frequency of the characteristic frequencies.

10. The apparatus (36) according to claim 8 or 9, further comprising
a flavour playing back unit for playing back at least one flavour allotted to the played back frequency of the characteristic frequencies.

11. The method according to one of claims 1 to 7, wherein the steps of the method are formulated as a program code by means of which the method runs on a computer.

## Revendications

1. Procédé de rendu sonore individuel et ciblé sur une
personne, présentant les étapes consistant à : enregistrer une voix humaine sous forme électronique, déterminer un histogramme pour représenter
l'occurrence relative d'une fréquence fondamentale
déterminée dans la voix enregistrée, analyser l'histogramme pour identifier trois
fréquences caractéristiques, mémoriser les trois fréquences caractéristiques sous
forme électronique, et restituer un son avec l'une des trois fréquences
caractéristiques déterminées sous forme acoustique.

2. Procédé selon la revendication 1, dans lequel les trois fréquences caractéristiques sont déterminées sous forme d'amplitudes dans l'histogramme de la voix humaine.

3. Procédé selon la revendication 1 ou 2, dans lequel les trois fréquences caractéristiques sont distantes l'une de l'autre.

4. Procédé selon la revendication 3, dans lequel les trois fréquences caractéristiques se présentent sous la forme d'une fréquence fondamentale, d'une fréquence de position indifférente et d'une fréquence vitale, la fréquence de position indifférente présentant une fréquence qui est sensiblement supérieure d'une tierce mineure ou d'une tierce majeure à la fréquence de la fréquence fondamentale et la fréquence vitale présente une fréquence qui est sensiblement supérieure d'une tierce majeure ou d'une quarte majeure à la fréquence de la fréquence de position indifférente.

5. Procédé selon l'une quelconque des revendications précédentes, contenant en outre les étapes consistant à :
affecter des teintes correspondantes (40) aux fréquences caractéristiques déterminées et
restituer visuellement la teinte affectée (40) conjointement avec l'une des trois fréquences caractéristiques.

6. Procédé selon la revendication 5, dans lequel les fréquences des teintes (40) affectées aux fréquences caractéristiques sont d'autant plus élevées que les fréquences des fréquences caractéristiques sont élevées.

7. Procédé selon l'une quelconque des revendications précédentes, comprenant en outre l'étape visant à affecter des aromes correspondants aux fréquences caractéristiques déterminées.

8. Dispositif (10) pour mettre en oeuvre le procédé selon l'une quelconque des revendications 1 à 4, comprenant :
une unité d'enregistrement (12) pour enregistrer une voix humaine,
une unité de calcul (14) pour déterminer un histogramme pour représenter l'occurrence relative d'une fréquence fondamentale déterminée dans la voix enregistrée,
une unité d'analyse (16, 38) pour analyser l'histogramme pour identifier trois fréquences caractéristiques,
une unité de mémorisation (18) pour mémoriser les trois fréquences caractéristiques sous forme électronique, et
une unité de restitution (20) pour restituer un son avec l'une des trois fréquences caractéristiques.

9. Dispositif (36) selon la revendication 8, comprenant en outre :
une unité d'analyse (38) pour attribuer une teinte (40) aux fréquences caractéristiques identifiées, et
une unité de restitution de teinte (42) pour restituer la teinte (40) qui est attribuée à la fréquence restituée des fréquences caractéristiques.

10. Dispositif (36) selon la revendication 8 ou 9, comprenant en outre :
une unité de restitution d'aromes pour restituer au moins un arome qui est affecté à la fréquence restituée des fréquences caractéristiques.

11. Procédé selon l'une quelconque des revendications 1 à 7, dans lequel les étapes du procédé sont formulées en code de programme, au moyen duquel le procédé fonctionne sur un ordinateur.
